# EUROPEAN PATENT APPLICATION

(11) **EP 1 698 329 A1**
(43) Date of publication of application: **06.09.2006**
(21) Application number: 04792270.3
(22) Date of filing: 12.10.2004
(51) Int. Cl.: A61K 9/14, A61K 9/06, A61K 9/08, A61K 9/10, A61K 9/20, A61K 9/70, A61K 9/72, A61K 47/02, A61K 47/04, A61K 47/10, A61K 47/12, A61K 47/24, A61K 47/34, A61K 47/36, A61K 31/07, A61K 31/122, A61K 31/198, A61P 3/02

(54) **DRUG-CONTAINING NANOPARTICLE, PROCESS FOR PRODUCING THE SAME AND PARENTERALLY ADMINISTERED PREPARATION FROM THE NANOPARTICLE**

(30) Priority: 24.12.2003 JP 2003428462
(71) Applicant: LTT Bio-Pharma Co., Ltd., Tokyo 105-6201 (JP)
(72) Inventor: ISHIHARA, Tsutomu, 1430024 (JP); MIZUSHIMA, Yutaka, 1060032 (JP); SUZUKI, Jun;, Saitama-shi, Saitama 336-0024 (JP); SEKINE, Junzou, 3600013 (JP); YAMAGUCHI, Yoko,, Ashigarakami-gun, Kanagawa 258-0022 (JP); IGARASHI, Rie, Kawasaki-shi, Kanagawa 21-40036 (JP)
(74) Representative: Albrecht, Thomas
(86) International application number: PCT/JP2004/015026
(87) International publication number: WO 2005/060935

(57) **Abstract**

To provide an external preparation or injectable preparation that exerts the effect of enabling transdermal or transmucosal in vivo absorption of fat-soluble drugs and water-soluble drugs not having been satisfactorily attained hitherto and that contains a highly absorbable fat-soluble/water-soluble drug, the injectable preparation especially aiming at sustained-release and target effects.

In particular, drug-containing nanoparticles (secondary nanoparticles) are provided by causing primary nanoparticles containing a fat-soluble drug or fat-solubilized water-soluble drug to act with a bivalent or trivalent metal salt. Further, drug-containing nanoparticles (tertiary nanoparticles) are provided by first causing primary nanoparticles containing a fat-soluble drug or fat-solubilized water-soluble drug to act with a bivalent or trivalent metal salt to thereby obtain secondary nanoparticles and thereafter causing a monovalent to trivalent basic salt to act on the secondary nanoparticles. Still further, there are provided a process for producing these nanoparticles, and a transdermal or transmucosal external preparation or injectable preparation in which these nanoparticles are contained.

## Description

### TECHNICAL FIELD

The present invention relates to nanoparticles containing a fat-soluble drug or fat-solubilized water-soluble drug, and more specifically to nanoparticles of a fat-soluble drug or fat-solubilized water-soluble drug and a process for producing the same, and parenteral preparations for transdermal or transmucosal application and for injection comprising the nanoparticles.

### BACKGROUND ART

The purpose of transdermal or transmucosal administration of drugs is to mitigate the defects associated with oral preparations, for example, (1) poor drug absorption through gastrointestinal tract causes nonuniform absorption and inactivation in liver, (2) rapid drug absorption causes a side effect which is particularly strong in gastrointestinal tracts and liver, and (3) sustained release of drug is not attained.

As to transdermal or transmucosal application of drug, a plenty of techniques have been brought into practical use. Such techniques have the problems that absorption and distribution to skin or mucosa and permeation to subcutaneous and submucosa tissues are insufficient when such techniques intend for local effect, and that insufficient systemic absorption is observed in a considerable number of drugs when such drugs are intended for systemic absorption.

As to external preparations intended for systemic administration of drugs, those cause side effects at epidermis or mucosa, and those inactivated by metabolic enzymes of skin or mucosa to be converted to substances having side effects are known, and in these cases, the drugs need to be passed through skin tissues without being reacted or metabolized in epidermis or mucosa. For example, transdermal preparations of testosterone are widely used, however, it is known that considerable part of testosterone is metabolized into an active metabolite that causes hair loss or prostatic cancer by 2,5-dihydroxynase which is present in the skin.

For bioactive proteins and peptides that are inactivated during oral administered and hence necessitate administration by injection, attempts have been made to administer in a transdermal or transmucosal route in recent years. However, it is still impossible to ensure improvement of the absorption.

Various researches for transdermal or transmucosal administration method are undertaken using insulin which is relatively low in molecular weight and chemically stable as one of bioactive proteins. However, absorption of insulin is only several percentages according to reliable data and little insulin is absorbed in the case of transdermal administration (Non-patent document 1).

Also proposed are preparations produced by encapsulating a bioactive substance in calcium-containing low-water soluble inorganic particles (Patent document 1), and a water-insoluble sustained-release composition comprising precipitates formed of a bioactive protein or peptide and zinc ion (Patent document 2). These preparations, however, are not still satisfactory in terms of drug absorption and local stimulation, and hence have not been brought into practical use. The techniques that aims at transdermal or transmucosal *in vivo* absorption using nanoparticles containing a fat-soluble drug or fat-solubilized water-soluble drug as is intended by the present invention is not known heretofore.

Patent document 1: International Publication No. WO 02/096396 Patent document 2: Japanese Patent Laid-Open Publication No. 2003-081865
Non-patent document 1: DRUG DELIVERY SYSTEM "Today's DDS drug delivery system (Iyaku (medicine and Drug) Journal) pp.325-331, 1999.
Non-patent document 2: Clinical Pharmacology (Jpn. J. Clin. Pharmacol. Ther.,): 26(1), p.127-128(1995)
Non-patent document 3: Yakugaku Zasshi: 121(12), p.929-948(2001)
Non-patent document 4: J. Controlled Release: 79, p.81-91(2002)

As described above, there is a demand for development of preparations, which enable drugs that are little absorbed or inactivated or give side effects when orally administered, to be administered in a transdermal or transmucosal route, and which ensure excellent absorption of drugs and adequate exertion of activity and least side effects.

### DISCLOSURE OF INVENTION

### PROBLEMS TO BE SOLVED BY THE INVENTION

Therefore, it is an object of the present invention to provide a technique that imparts high in vivo (including local) absorptivity and high bioavailability by transdermal and transmucosal administration methods, to the drugs that fail to exert the drug efficacy when orally administered or have drawbacks in absorptivity, side effect and the like, and to the drugs that are used as injectable agents or skin external preparations, but need improvement in absorptivity, side effect and the like.

In order to achieve the above object, the present inventors made diligent efforts and succeeded in making special nanoparticles that are much smaller than erythrocytes be contained in a drug by applying nanotechnology, and found that when such nanoparticles are administered transdermally or transmucosally, the drug contained in the nanoparticles is well absorbed in vivo and excellent in bioavailability, and finally accomplished the present invention.

The inventors previously invented nanoparticles of bioactive proteins or peptides, and made an application for patent (Japanese patent application No. 2003-312031). The inventors succeed in preparation of nanoparticles for fat-soluble drugs and water-soluble drugs other than proteins and peptides, and accomplished the present invention.

### MEANS FOR SOLVING THE PROBLEM

Therefore, the present invention provides nanoparticles containing a fat-soluble drug or water-soluble drug, exhibiting excellent absorptivity and bioavailability when administered through skin or mucosa for the purpose of systemic administration and local administration. Nanoparticles of the present invention may also be advantageously used as injectable agents.
More specifically, the present invention provides:
(1) Drug-containing nanoparticles provided by causing primary nanoparticles containing a fat-soluble drug or a fat-solubilized water-soluble drug to act with a bivalent or trivalent metal salt;
(2) Drug-containing nanoparticles provided by causing primary nanoparticles containing a fat-soluble drug or fat-solubilized water-soluble drug to act with a bivalent or trivalent metal salt to give secondary nanoparticles, and causing a monovalent to trivalent basic salt to act with the secondary nanoparticles;
(3) The drug-containing nanoparticles according to the above (1) or (2), wherein the primary nanoparticles are produced by causing the fat-soluble drug or the fat-solubilized water-soluble drug, a medium- or long-chain organic compound having a negative ion residue and a surfactant to act with each other;
(4) The drug-containing nanoparticles according to the above (3), wherein the medium- or long-chain organic compound having a negative ion residue is a C₆-C₂₄ fatty acid or its salt;
(5) The drug-containing nanoparticles according to the above (4), wherein the C₆-C₂₄ fatty acid is selected from unsaturated fatty acids such as oleic acid, linoleic acid, and linolenic acid, and saturated fatty acids such as lauric acid, myristic acid, and palmitic acid;
(6) The drug-containing nanoparticles according to the above (1) or (2), wherein the bivalent or trivalent metal salt is a calcium salt, a zinc salt, an iron salt, or a copper salt;
(7) The drug-containing nanoparticles according to the above (2), wherein the monovalent to trivalent basic salt is selected from hydrogen carbonates, hydrogen phosphates, carbonates, phosphates, oxalates, lactates, and urates;
(8) The drug-containing nanoparticles according to the above (1) or (2), wherein fat-solubilization of water-soluble drug is carried out by contact between the water-soluble drug and the bivalent or trivalent metal ion, contact between the water-soluble drug and an acidic or basic polysaccharide, or adjustment of pH or change in ion strength of the solution in which the water-soluble drug is dissolved;
(9) The drug-containing nanoparticles according to the above (8), wherein the bivalent or trivalent metal ion to be brought into contact with the water-soluble drug is selected from a zinc ion, a calcium ion, an iron ion, and a copper ion;
(10) The drug-containing nanoparticles according to the above (3) or (9), wherein the surfactant is one or more selected from glycerin, lecithin, polyoxyethylene (20) sorbitan monooleate (Tween 80), polyoxyethylene (20) sorbitan monolaurate (Tween 20), polyoxyethylene (20) sorbitan monostearate (Tween 60), polyoxyethylene (20) sorbitan monoparmitate (Tween 40), polyoxyethylene (20) sorbitan trioleate (Tween 85), polyoxyethylene (8) octylphenyl ether, polyoxyethylene (20) cholesterol ester, lipid-polyethylene glycol, polyoxyethylene hydrogenated castor oil, and fatty acid-polyethylene glycol copolymer;
(11) The drug-containing nanoparticles according to any one of the above (1) to (10), wherein the fat-soluble drug or water-soluble drug is a chemical compound that has a molecular weight of 1000 or less, exhibits bioactivity and are applicable to human;
(12) The drug-containing nanoparticles according to the above (11), wherein the fat-soluble drug is insoluble to poorly soluble to water and soluble to organic solvents;
(13) The drug-containing nanoparticles according to the above (11) or (12), wherein the fat-soluble drug is selected from steroid hormones, immuno suppressing or modulating agents, anticancer agents, antibiotics, chemotherapeutic agents, antiviral agents, non-steroidal anti-inflammatory agents, antipsychotic agents, calcium antagonists, antihypertensive agents, prostaglandin drugs, and lipophilic vitamins;
(14) The drug-containing nanoparticles according to any one of the above (11) to (13), wherein the fat-soluble drug is selected from testosterone enanthate, testosterone propionate, testosterone, estradiol, estradiol valerate, estradiol benzoate, dexamethasone acetate, betamethasone, betamethasone dipropionate, betamethasone valerate, prednisolone acetate, cyclosporine, tacrolimus, paclitaxel, irinotecan hydrochloride, cisplatin, methotrexate, carmofur, tegafur, doxorubicin, clarithromycin, aztreonam, cefdinir, nalidixic acid, ofloxacin, norfloxacin, ketoprofen, flurbiprofen, flurbiprofen axetil, chlorpromazine, diazepam, nifedipine, nicardipine hydrochloride, amlodipine besilate, candesartan cilexetil, aciclovir, vidarabine, efavirenz, alprostadil, dinoprostone, ubidecarenone, vitamin A (retinol), vitamin D, vitamin E, and vitamin K;
(15) The drug-containing nanoparticles according to the above (11), wherein the water-soluble drug is a drug that is fat-solubilized by binding with a bivalent or trivalent metal ion;
(16) The drug-containing nanoparticles according to the above (11) or (15), wherein the water-soluble drug is selected from water-soluble steroid hormones, immuno suppressing or modulating agents, anticancer agents, antibiotics, chemotherapeutic agents, antiviral agents, non-steroidal anti-inflammatory agents, antipsychotic agents, antihypertensive agents, prostaglandin drugs, and vitamins;
(17) The drug-containing nanoparticles according to the above (11), (15), or (16), wherein the water-soluble drug is selected from betamethasone phosphate, dexamethasone phosphate, hydrocortisone phosphate, prednisolone phosphate, prednisolone succinate, hydrocortisone succinate, vancomycin, vincristine, vinplastin chloramphenicol succinate, latamoxef, cefpirome, carumonam, clindamycin phosphate, and abacavir;
(18) The drug-containing nanoparticles according to the above (11), wherein the fat-soluble drug is testosterone enanthate, cyclosporine, betamethasone valerate, ubidecarenone or vitamin A (retinol), and the water-soluble drug is betamethasone phosphate; and
(19) The drug-containing nanoparticles according to any one of the above (1) to (18), wherein the particles have a diameter ranging from 1 to 150 nm.

Further, the present invention provides:
(20) A transdermal or transmucous external preparation comprising the drug-containing nanoparticles according to any one of the above (1) to (19);
(21) The external preparation according to the above (20), wherein the external preparation is selected from ointments, gels, sublingual tablets, buccal tablets, liquids and solutions, sprays for buccal/lower respiratory tract, inhalations, suspensions, hydrogels, lotions, cataplasms, and patches;
(22) An injectable preparation comprising the drug-containing nanoparticles according to any one of the above (1) to (19);
(23) A process of producing drug-containing nanoparticles comprising; dissolving a fat-soluble drug or fat-solubilized water-soluble drug, a medium- or long-chain organic compound having a negative ion residue, and a surfactant in an organic solvent or a water-containing organic solvent to give a solution; dispersing the solution in water to produce primary nanoparticles; and causing a bivalent or trivalent metal salt to act with the solution containing the primary nanoparticles;
(24) A process of producing drug-containing nanoparticles comprising; dissolving a fat-soluble drug or fat-solubilized water-soluble drug, a medium- or long-chain organic compound having a negative ion residue and a surfactant in an organic solvent or a water-containing organic solvent to give a solution; dispersing the solution in water to produce primary nanoparticles; causing a bivalent or trivalent metal salt to act with the solution containing the primary nanoparticles to produce secondary nanoparticles; and causing a monovalent to trivalent basic salt to act with the secondary nanoparticles;
(25) The production process according to the above (23) or (24), wherein the organic solvent is one or more selected from acetone, ethanol, propanol, and butanol; and
(26) The production process according to the above (23) or (24), wherein fat-solubilization of the water-soluble drug comprises bringing the water-soluble drug into contact with the bivalent or trivalent metal ion.

### EFFECT OF THE INVENTION

Nanoparticles provided by the present invention allows transdermal or transmucosal in vivo absorption of the fat-soluble drug and the water-soluble drug contained therein, and achieves excellent sustained-releasability and targeting when administered by injection. Therefore, the present invention revolutionarily enables transdermal or transmucosal *in vivo* absorption of fat-soluble drugs and the water-soluble drugs, that has not been achieved satisfactorily, and provides external preparations and injectable agents containing a fat-soluble or water-soluble drug and having excellent absorptivity and sustained-releasability. The nanoparticles of the present invention, when transdermally administered, permeate from the epidermis to deep parts and distribute at high concentrations in dermal and subcutaneous tissues, therefore, they are very useful for diseases in joints, peritenons, and muscles near skin. This also applies to submucosal tissues, and applications to varied diseases are possible. Further, drugs which are physiochemically unstable may be greatly stabilized by making the nanoparticles of the present invention. Therefore, the nanoparticles of the present invention also have applications to pharmaceuticals, medicated cosmetics, and cosmetics.

### BEST MODE FOR CARRYING OUT THE INVENTION

As described above, the present invention relates to drug-containing nanoparticles (secondary nanoparticles) provided by causing primary nanoparticles containing a fat-soluble drug or a fat-solubilized water-soluble drug to act with a bivalent or trivalent metal salt, and drug-containing nanoparticles (tertiary nanoparticles) provided by causing primary nanoparticles containing a fat-soluble drug or fat-solubilized water-soluble drug to act with a bivalent or trivalent metal salt to thereby obtain secondary nanoparticles and thereafter causing a monovalent to trivalent basic salt to act on the secondary nanoparticles, as well as a process for producing these nanoparticles, and a transdermal or transmucosal external preparation or injectable preparation in which these nanoparticles are contained.

Nanoparticles according to the present invention have a particle size of approximately 1 to 200 nm, preferably approximately 5 to 150 nm in diameter. Such a particle size may be adjusted depending on the blending ratio of a drug to be contained and a medium- or long-chain organic compound, adding amount of surfactant, adding amount of monovalent to trivalent basic salt, amount of used solvent, strength of stirring and the like, and particles having a diameter of approximately 5 to 500 nm may be prepared. The particle size increases with the amount of surfactant, however, too small amount of surfactant causes aggregation of particles and formation of large particles. Particle size may be determined by a light scattering method or electron microscopic measurement.

As the fat-soluble drug contained in the nanoparticles provided by the present invention, any drugs that are insoluble to poorly soluble to water and soluble to organic solvents can be used, and such drugs are selected from, for example, steroid hormones, immuno suppressing or modulating agents, anticancer agents, antibiotics, chemotherapeutic agents, antiviral agents, non-steroidal anti-inflammatory agents, antipsychotic agents, calcium antagonists, antihypertensive agents, prostaglandin drugs, and lipophilic vitamins. More specific examples include, but are not limited to, testosterone enanthate, testosterone propionate, testosterone, estradiol, estradiol valerate, estradiol benzoate, dexamethasone acetate, betamethasone, betamethasone dipropionate, betamethasone valerate, prednisolone acetate, cyclosporine, tacrolimus, paclitaxel, irinotecan hydrochloride, cisplatin, methotrexate, carmofur, tegafur, doxorubicin, clarithromycin, aztreonam, cefdinir, nalidixic acid, ofloxacin, norfloxacin, ketoprofen, flurbiprofen, flurbiprofen axetil, chlorpromazine, diazepam, nifedipine, nicardipine hydrochloride, amlodipine besilate, candesartan cilexetil, aciclovir, vidarabine, efavirenz, alprostadil, dinoprostone, ubidecarenone, vitamin A (retinol), vitamin D, vitamin E, and vitamin K.

When the above drugs have their salt, ester, stereoisomer, enantiomer, solvate, and the like, all of such substances are also embraced.

The water-soluble drug contained in the nanoparticles provided by the present invention may be any drugs insofar as they bind to a bivalent or trivalent metal ion to thereby be fat-solubilized can be used, and is selected from, for example, water-soluble steroid hormone, immuno suppressing or modulating agents, anticancer agents, antibiotics, chemotherapeutic agents, antiviral agents, non-steroidal anti-inflammatory agents, antipsychotic agents, antihypertensive agents, prostaglandin drugs, and vitamins, and is preferably a drug having an intramolecular phosphoric group, carboxyl group or sulfate group. More preferred examples include, but are not limited to, betamethasone phosphate, dexamethasone phosphate, prednisolone phosphate, prednisolone succinate, hydrocortisone succinate, vancomycin, vinplastin, vincristine, chloramphenicol succinate, latamoxef, cefpirome, carumonam, clindamycin phosphate, and abacavir.

When the above drugs have their salt, ester, stereoisomer, enantiomer, solvate, and the like, all of such substances are also embraced.

In production of the primary nanoparticles of the present invention, a fat-soluble drug or a water-soluble drug need to be fat-solubilized. Most preferred means for fat-solubilizing a water-soluble drug is to use a bivalent or trivalent metal ion that forms a precipitate with the water-soluble drug. Examples of such bivalent or trivalent metal ion include zinc ions from zinc salts such as zinc acetate, zinc chloride and zinc sulfate; calcium ions from calcium salts such as calcium carbonate, calcium chloride and calcium sulfate; iron ions from iron salts such as iron chloride and iron sulfide; and copper ions from copper-salts such as copper chloride and copper sulfate, and among these, zinc ions are preferably used.

In this case, the blending ratio between the water-soluble drug and the bivalent or trivalent metal ion is not particularly limited, and may be any ratios that allows generation of a precipitate due to binding of these substances. In the case of zinc ion, for example, the water-soluble drug and the zinc salt may be blended in a ratio of about 10:1 to 1:10 by weight ratio. Fat-solubilization may be achieved by contacting with acidic or basic polysaccharides such as sodium chondroitin sulfate, hyaluronan, and chitosan, or adjusting pH or changing ion strength of solution in which the water-soluble drug is dissolved. The fat-soluble drug may be used as it is.

In order to produce primary nanoparticles of the present invention, an organic compound having a negative ion residue such as carboxyl group, phosphoric group, sulfate group or the like is required, and as such an organic compound, any compounds having such a residue are applicable, however, a medium- or long-chain organic compound having a carboxyl group is particularly preferred. As such a medium- or long-chain organic compound having a negative ion residue, C₆-C₂₄ unsaturated or saturated fatty acids or their salts are preferred, and unsaturated fatty acids such as oleic acid, linoleic acid and linolenic acid and saturated fatty acids such as lauric acid, myristic acid and palmitic acid are preferred, and oleic acid and myristic acid are particularly preferred. When such a medium- or long-chain organic compound is powder, it may be added as it is, but preferably dissolved in water, an organic solvent or water-containing organic solvent before use. As such an organic solvent, acetone, methanol, ethanol, propanol, butanol, and the like lower alcohols can be used, and among these, acetone and ethanol are preferred. Preferably, the blending ratio between the fat-soluble drug or fat-solubilized water-soluble drug and the medium- or long-chain organic compound is about 1:30 to 1:0.03.

In this production step of primary nanoparticles, a stirrer or an ultrasonic wave generator is used for obtaining desired uniform condition containing fine particles, and by raising the pressure using a French presser, a Mantle goaly or the like, primary nanoparticles are produced as finer nanoparticles.

In production of the primary nanoparticles of the present invention, for the purpose of preventing the generated nanoparticles from aggregating, an appropriate amount of surfactant is preferably added, and the adding amount may be appropriately selected so that the nanoparticles do not aggregate each other, and preferably such a surfactant is used in a molar ratio of about 0.3 to 0.01 relative to the medium- or long-chain organic compound. As such a surfactant, glycerin, lecithin, polyoxyethylene (20) sorbitan monooleate (Tween 80), polyoxyethylene (20) sorbitan monolaurate (Tween 20), polyoxyethylene (20) sorbitan monostearate (Tween 60), polyoxyethylene (20) sorbitan monoparmitate (Tween 40), polyoxyethylene (20) sorbitan trioleate (Tween 85), polyoxyethylene (8) octylphenyl ether, polyoxyethylene (20) cholesterol ester, lipid-polyethylene glycol, fatty acid-polyethylene glycol, polyoxyethylene hydrogenated castor oil, fatty acid-polyethylene glycol copolymer, and the like nonionic surfactants can be used. One or more of these surfactants may be selected and used. Among these, glycerin, lecithin, polyoxyethylene (20) sorbitan monooleate (Tween 80), polyoxyethylene (20) sorbitan monolaurate (Tween 20), and fatty acid-polyethylene glycol copolymer are preferred, and as a fatty acid in this case, unsaturated fatty acids such as oleic acid, linoleic acid, and linolenic acid, and saturated fatty acids such as lauric acid, myristic acid, and palmitic acid can be exemplified.

Furthermore, vegetable oil may be added. As the vegetable oil used in this case, vegetable oils such as soybean oil, sesame oil, corn oil, olive oil and various salad oils can be preferably used.

Nanoparticles of the present invention include secondary nanoparticles provided by causing the primary nanoparticles obtained in the manner as described above to act with a bivalent or trivalent metal salt, and tertiary nanoparticles provided by causing the secondary nanoparticles to act with a monovalent to trivalent basic salt.

The bivalent or trivalent metal salt used herein is for example, calcium salts such as calcium chloride, calcium acetate and calcium sulfate; zinc salts such as zinc acetate, zinc chloride and zinc sulfate; iron salts such as iron chloride and iron sulfide; or copper salts such as copper chloride and copper sulfide, and calcium salts, especially calcium chloride is preferred among these. The blending amount of metal salt is not particularly limited, but it may preferably be used in a weight ratio of about 5 to 0.01 relative to the drug which is an active ingredient.

As the monovalent to trivalent basic salt for obtaining tertiary particles, hydrogen carbonates such as sodium hydrogen carbonate and potassium hydrogen carbonate; hydrogen phosphates such as sodium hydrogen phosphate and potassium hydrogen phosphate; carbonates such as sodium carbonate, potassium carbonate and calcium carbonate; phosphates such as sodium phosphate, potassium phosphate and calcium phosphate; oxalates such as sodium oxalate, potassium oxalate and calcium oxalate; lactates such as sodium lactate, potassium lactate and calcium lactate; and urates such as sodium urate, potassium urate and calcium urate can be exemplified, and among these, hydrogen carbonates and carbonates are preferred in the case of the fat-soluble drug, and carbonates. Especially sodium carbonate is preferred in the case of fat-solubilized water-soluble drug. The blending amount of the basic salt is not particularly limited, but it may preferably be used in a molar ratio of about 1.0 to 0.05 relative to the bivalent or trivalent metal salt.

Next, a process for producing nanoparticles provided by the present invention will be explained.
First, a fat-soluble drug or fat-solubilized water-soluble drug, a medium- or long-chain organic compound having a negative ion residue, and a surfactant are dissolved in an organic solvent or in a water-containing organic solvent, and the resultant solution is dispersed in mass volume of water and stirred for about 1 to 30 minutes, to thereby produce primary nanoparticles. To the solution containing the primary nanoparticles thus produced is added a bivalent or trivalent metal salt, and the resultant solution is stirred for 1 to 30 minutes to produce secondary nanoparticles. Then to the solution containing the secondary nanoparticles thus obtained, is added a monovalent to trivalent basic salt, and the resultant solution is stirred for one minute to 24 hours, to give tertiary nanoparticles. The water-soluble drug may be fat-solubilized by dissolving the water-soluble drug in acidic, basic or neutral water, and adding to the resultant solution a bivalent or trivalent metal ion.

After removing solvents from the solutions of secondary nanoparticles and tertiary nanoparticles containing a fat-soluble drug or fat-solubilized water-soluble drug thus produced according to the present invention by freeze-drying, reduced-pressure drying, or spray-drying, a transdermal or transmucosal external preparation or injectable preparation which is a desired parenteral preparation can be prepared by using an appropriate formulation base, additive and the like as compositions for preparation.

The present invention also provides such a transdermal or transmucosal external preparation or injectable preparation. Such an external preparation may be administered systemically or locally for therapeutic purpose in various forms including application, patch, and spray, and concrete examples of such an external preparation include ointments, gels, sublingual tablets, buccal tablets, liquids and solutions, sprays for buccal/lower respiratory tract, inhalations, suspensions, hydrogels, lotions, cataplasms and patches. Liquids and solutions are suited for nasal drops and ophthalmic solutions. Also application to skin or mucosa, and spray to lower respiratory tract are effective administration forms. Injectable preparations may be administered by any of intravenous, subcutaneous, muscle injections which are selected depending on the characteristic of particular drugs.

As bases and other additive components used in preparing these external preparations or injectable preparations, bases and components that are used in preparation of external preparations or injectable preparations in the pharmaceutical field can be exemplified. Concrete examples include oleaginous bases such as vaseline, plustibase, paraffin, liquid paraffin, light liquid paraffin, white beeswax and silicon oil; vehicles such as water, water for injection, ethanol, methylethylketone, cotton seed oil, olive oil, peanut oil and sesame oil; nonionic surfactants such as polyoxyethylene hydrogenated castor oil, polyoxyethylene sorbitan fatty acid ester, glycerin fatty acid ester, polyoxyethylene fatty acid ester, polyoxyethylene alkyl ether, sorbitan fatty acid ester and polyoxyethylene polyoxypropylene glycol; viscosity-increasing agents such as polyvinylpyrrolidone, sodium carboxymethyl cellulose (CMC), xanthan gum, tragacanth gum, gum arabic, gelatin and albumin; stabilizers such as dibutylhydroxytoluene; humecants such as glycerin, 1,3-butyleneglycol, propyleneglycol, urea, sucrose, erythritol and sorbitol; antiseptic agents such as methyl paraoxybenzoate, buthyl paraoxybenzoate, sodium dehydroacetate and p-cresol, which may be appropriately selected and used depending on the dose form. In the case of nasal drops, a nasal absorption promoting agent such as hydropropyl cellulose is preferably blended. For producing hydrogels, gelators such as sodium carboxymethyl cellulose (CMC), methylcellulose, hydroxymethyl cellulose, and polyvinylpyrrolidone are used.

For example, in the case of an ointment containing nanoparticles of the present invention as an active ingredient, vaseline is preferably used as a component of bases and the like, together with 0.05 to 0.5% of sodium carboxymethyl cellulose (CMC) for stabilizing the suspension.

### [Examples]

The invention will be explained in more detail with reference to the following examples and test examples, however, the present invention is not limited to these.

### Example 1: Preparation of secondary particles- Effect of surfactant

10 mg of sodium oleate was added to 0.1 mL of water, and thoroughly dissolved to form micelle by using an ultrasonic bath. Then 1 mg of testosterone enanthate or 1 mg of cyclosporine A dissolved in predetermined amounts of Tween 80 and ethanol is added and mixed to uniformity for 10 minutes using an ultrasonic wave generator. Then a predetermined amount of calcium chloride aqueous solution was added and stirred for 30 minutes, to produce secondary nanoparticles containing testosterone enanthate or cyclosporine A. The solution containing a drug thus obtained was then centrifuged at 10,000 rpm for 10 minutes, and testosterone enanthate and cyclosporine A contained in the supernatant were quantified by HPLC. The results are shown in Tables 1 and 2.

Effect of amounts (weight ratio) of calcium and Tween on formation of particles containing testosterone enanthate (TE)

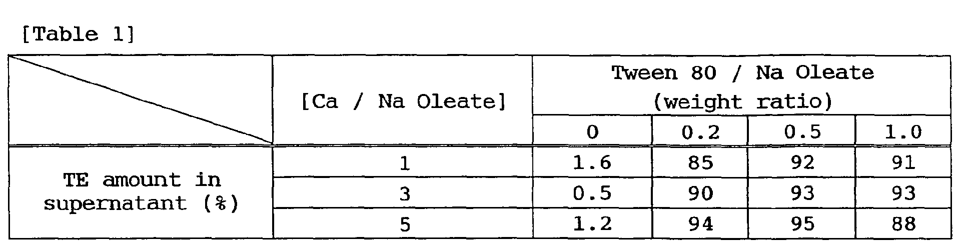

Effect of amounts (weight ratio) of calcium and Tween on formation of particles containing cyclosporine A (CYA)

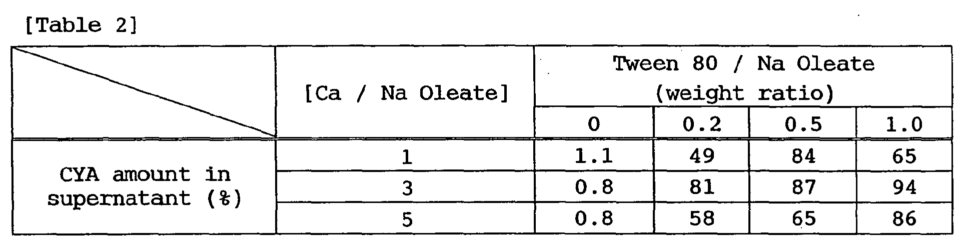

The results shown in Tables 1 and 2 demonstrated that large particles or aggregates were formed and the drug was deposited in the absence of Tween 80, and that small particles containing the drug were formed in the presence of 2 mg or more of Tween 80. The drug content was not influenced by change in calcium amount.

### Example 2: Preparation of secondary particles

10 mg of sodium oleate was added to 0.1 mL of water, and thoroughly dissolved to micelle by using an ultrasonic bath. Then 1 mg of betamethasone valerate dissolved in predetermined amounts of Tween 80 and ethanol was mixed, and then irradiated with ultrasonic waves for 10 minutes. Then 33 µL of 1M calcium chloride aqueous solution was added, and stirred for 30 minutes, to thereby produce secondary nanoparticles containing betamethasone valerate. The solution containing a drug thus obtained was then centrifuged at 10,000 rpm for 10 minutes, and contents of betamethasone valerate in the supernatant were quantified by HPLC. The results are shown in Table 3.

Particle formation of betamethasone valerate (BV)

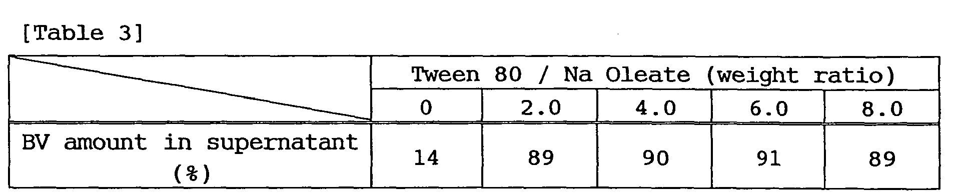

### Example 3: Relation between surfactant and particle size

To 10 mg of sodium oleate, a predetermined amount of lipid-PEG (phosphatidyl ethanolamine-PEG (MW: 2,000), product of NOF Corporation) or Tween 80 was mixed, and homogenized using an ultrasonic wave generator, and then 33 µL of 1M calcium chloride aqueous solution was added and the particle size was measured. The results are shown in Table 4.

Effect of amount of surfactant on particle size of surfactant/oleic acid particles

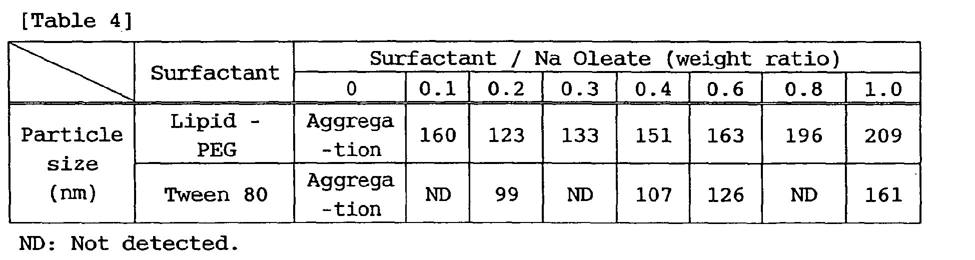

The result shown in Table 4 demonstrated that the larger the amount of surfactant, the larger the particle size became, and too small amount caused aggregation and formation of large particles, and there was a mixing ratio that realized the minimum particle size.

### Example 4: Preparation of tertiary nanoparticles - Effect of kind of metal salt/basic salt

10 mg of sodium oleate was added to 0.1 mL of water, and thoroughly dissolved to micelle by using an ultrasonic bath. Then 6 mg of Tween 80 and 1 mg of cyclosporine A dissolved in ethanol were mixed and homogenized for 10 minutes using an ultrasonic wave generator. Then 1M calcium chloride or 1M zinc chloride was added in an equimolar amount relative to sodium oleate, and stirred for 30 minutes, to produce secondary nanoparticles containing cyclosporine A. The solution containing the secondary nanoparticles was then added with sodium hydrogen carbonate, sodium carbonate, or sodium dihydrogen phosphate in an equimolar amount relative to the metal salt, and stirred for 1 hour, to produce tertiary nanoparticles containing the drug. Then centrifugation at 10,000 rpm for 10 minutes was conducted, and cyclosporine A contained in the supernatant was quantified by HPLC. The results are shown in Table 5.

Formation of tertiary nanoparticles using various kinds of metal salts and basic salts.

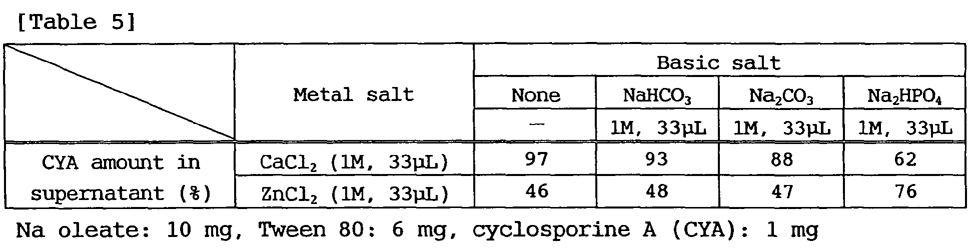

As is evident from the results of Table 5, smaller and more stable nanoparticles containing cyclosporine A were produced when calcium chloride was added, compared to the case where zinc chloride was added. When zinc chloride was added, smaller and more stable nanoparticles containing cyclosporine A were produced by using phosphate as a basic salt than using carbonate, and contrarily when calcium chloride was added, smaller and more stable nanoparticles containing cyclosporine A were produced by using carbonate as a basic salt than using phosphate.

### Example 5: Preparation of tertiary nanoparticles - Effect of basic salt

10 mg of sodium oleate was added to 0.1 mL of water, and thoroughly dissolved to micelle using an ultrasonic bath. Then 5 mg of Tween 80 and 1 mg of testosterone enanthate or 1 mg of cyclosporine A dissolved in ethanol were mixed, and homogenized for 10 minutes using an ultrasonic wave generator. Then calcium chloride was added in a molar ratio of three times the sodium oleate, followed by stirring for 30 minutes, to produce secondary nanoparticles containing testosterone enanthate or cyclosporine A. This solution containing secondary nanoparticles was then added with a predetermined amount of sodium hydrogen carbonate, and stirred for 1 hour, to produce tertiary nanoparticles containing a drug. Then centrifugation at 10,000 rpm for 10 minutes was conducted, and testosterone enanthate and cyclosporine A contained in the supernatant was quantified by HPLC. The results are shown in Tables 6 and 7.

Effect of amount of sodium hydrogen carbonate on formation of particles (testosterone enanthate, TE)

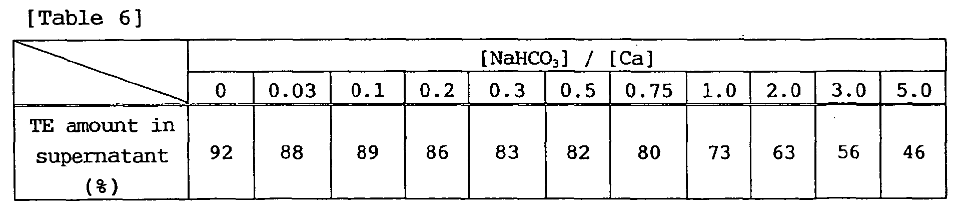

Effect of amount of sodium hydrogen carbonate on formation of particles (cyclosporine A, CYA)

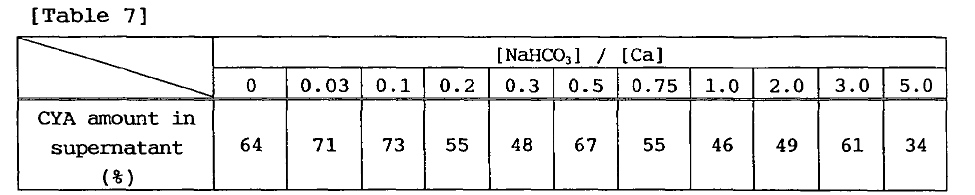

The results in Tables 6 and 7 demonstrated that the higher the proportion of sodium hydrogen carbonate relative to calcium, the smaller the amount of drug in the supernatant was. This is attributed to the fact that the excessively present carbonic acid reacts with calcium to form calcium carbonate, and the precipitates of the calcium carbonate and the prepared nanoparticles coprecipitate.

### Example 6: Preparation of tertiary nanoparticles

Tertiary nanoparticles containing testosterone enanthate coated with calcium phosphate were prepared in the same manner as described in Example 5 except that the drug in Example 5 was replaced by testosterone enanthate, and sodium hydrogen carbonate was replaced by disodium hydrogen phosphate (in an mount of 0.5 times by mole, relative to calcium chloride).

### Example 7: Preparation of tertiary nanoparticles - Effect of surfactant - Stability

10 mg of sodium oleate was added to 0.1 mL of water, and thoroughly dissolved to micelle by using an ultrasonic bath. Then Tween 80 which is a surfactant, a predetermined amount of polyoxyethylene cholesteryl ether (CS-20, available from Nihon Emulsion Co., Ltd.) or PEG-oleic acid (NOF Corporation), and 1 mg of cyclosporine A dissolved in ethanol were mixed, and homogenized for 10 minutes using an ultrasonic wave generator. Then 33 µL of 1M calcium chloride aqueous solution, and 16.5 µL of 1M sodium hydrogen carbonate aqueous solution were added sequentially under stirring, and then stirred for another hour, to thereby produce tertiary nanoparticles containing cyclosporine A. Then centrifugation at 10,000 rpm for 10 minutes was conducted, and the supernatant particulate suspension was added to water, brine, phosphate-buffered saline (PBS) or fetal bovine serum (FBS) in a volume ratio of 1:9, and absorbance at 550 nm was measured for evaluating stability in each solution. The results are shown in Table 8.

Dispersion stability of nanoparticles in each solution (turbidity change at 550 nm after 3 hours)

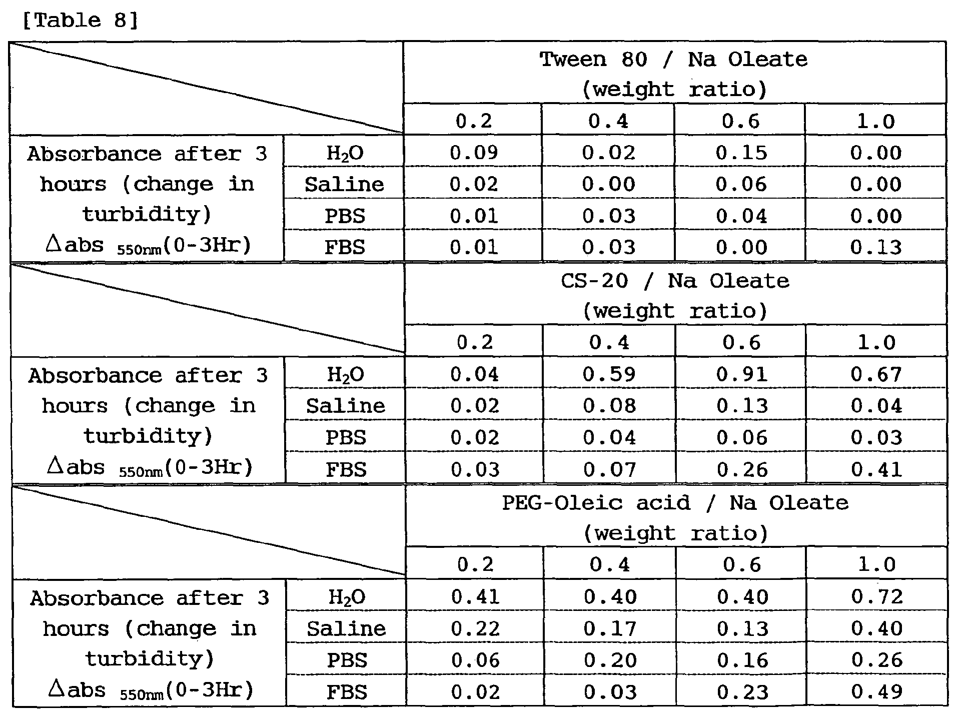

As is evident from the results shown in Table 8, nanoparticles comparable to those obtained by using Tween 80 were prepared by using CS-20 and PEG-oleic acid, however, nanoparticles prepared by using Tween 80 were most stable in each solution.

Also we measured particle sizes of particles in each preparation step of the present Example. The results are shown in Table 9 below. The results revealed that several hundreds nanometers of particles were obtained both in the secondary nanoparticles and the tertiary nanoparticles.

Change in particle size of nanoparticles

| | Na Oleate + Tween 80 | Tween 80 + Cacl₂ | Tween 80 + CaCl₂ + NaHCO₃ |
|---|---|---|---|
| Particle size (nm) | ND | 80 | 90 |

| | | | |
|---|---|---|---|
| ND: Not detected | | | |

### Example 8: Preparation of tertiary nanoparticles - Mouse transdermal absorption test

Using 10 mg of sodium oleate, 1 mg of cyclosporine A, 4 mg of Tween 80, 33 µL of 1M calcium chloride aqueous solution, and 16.5 µL of 1M sodium hydrogen carbonate aqueous solution, operations similar to those in Example 5 were conducted to produce tertiary nanoparticles containing cyclosporine A. The solution containing the nanoparticles thus obtained was centrifuged at 3,500 rpm to remove calcium carbonate precipitation, and then the supernatant was concentrated through Centriprep (YM-50, AMICON), to give tertiary nanoparticles containing cyclosporine A.

Cyclosporine A in the above particles was quantified by HPLC, and a particle suspension (25% glycerin aqueous solution) was applied to dehaired skin of back of 7-week-old ddy mouse such that the amount of cyclosporine A was 2 mg/animal. The same amount of particle suspension in water (without glycerin) was subcutaneously injected. As a reference example, the same amount of cyclosporine A (25% glycerin/50% ethanol aqueous solution) was applied, and whole blood were collected at 1, 3 and 24 hours from the administration and cyclosporine A contained in the plasma was determined by FPIA method. The results are shown in Table 10.

Changes in blood CYA concentration after application of cyclosporine A (CYA) encapsulating particles to mouse skin.

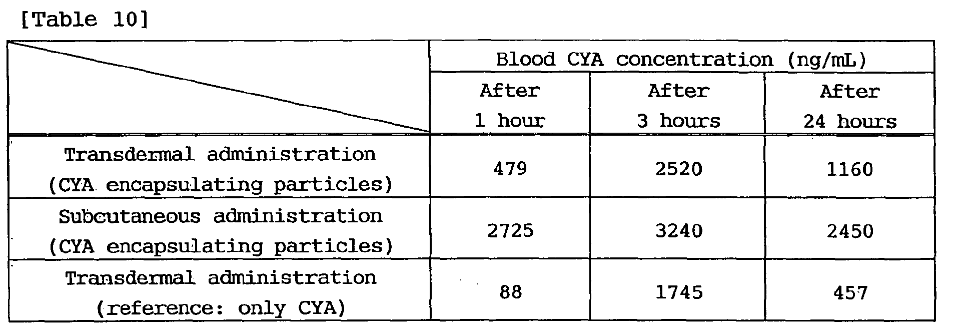

As is evident from the results shown in Table 10, when nanoparticles of the present invention were applied, higher blood concentration and sustained releasability were exhibited in comparison with the case where only cyclosporine A was applied. This demonstrates that making particles facilitates transdermal absorption cyclosporine A. Also when nanoparticles of the present invention were subcutaneously injected, high blood concentration was maintained even after 24 hours, and excellent absorptivity and sustained-releasability were observed.

### Example 9: Preparation of tertiary nanoparticles - Effects of use amounts of surfactant, fatty acid and solvent

In a predetermined amount of acetone, 10 mg of testosterone enanthate, a predetermined amount of myristic acid and Tween 80 were dissolved, and the resultant solution was added into water and stirred, to obtain primary particles containing testosterone enanthate. To this suspension of particles, was added 1M calcium chloride aqueous solution (equimolar amount relative to myristic acid) and stirred for 30 minutes to produce secondary particles. This solution was then added with 1M sodium hydrogen carbonate (0.2 times molar amount relative to calcium), and stirred for 1 to 12 hours. A solution containing tertiary nanoparticles prepared while changing the amounts of Tween 80, myristic acid and acetone appropriately was centrifuged at 10,000 rpm for 10 minutes, and particle size of the particles and amount of testosterone enanthate contained in the supernatant were determined. The results are shown in Tables 11 to 13.

Effect of amount of Tween on particle formation

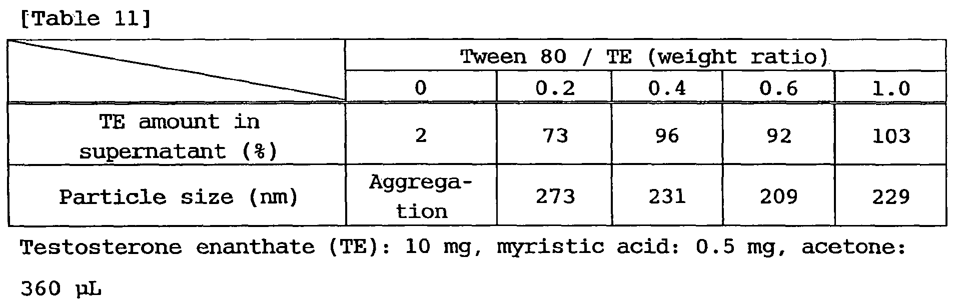

Effect of amount of myristic acid on particle formation

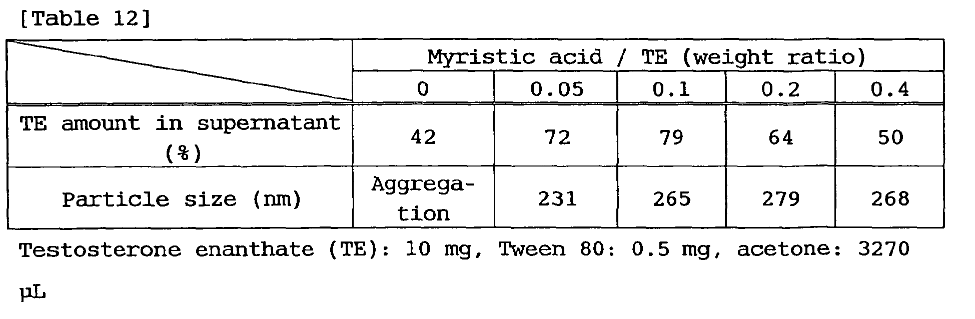

Effect of amount of acetone on particle formation

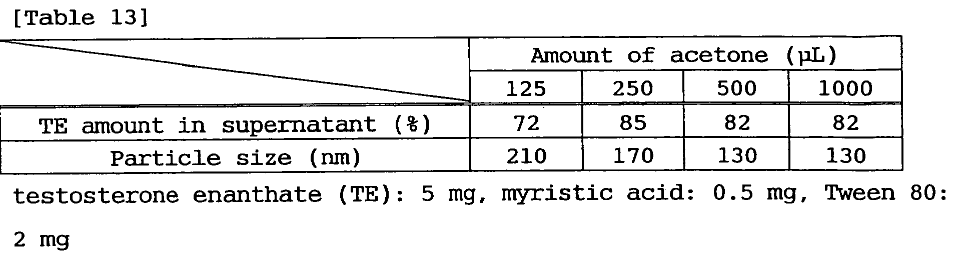

The results shown in Tables 11 to 13 demonstrated that the mixing ration between Tween 80 and drug, the mixing ratio between myristic acid and drug, and the use amount of acetone greatly influence on formation of particles (particle size and content of testosterone enanthate). It was also demonstrated that the larger the amount of Tween 80, the larger the content of testosterone enanthate was; the larger the mount of myristic acid, the smaller the content of testosterone enanthate was; and the larger the use mount of acetone, the smaller the particle size was.

### Example 10: Preparation of tertiary nanoparticles of water-soluble drug (betamethasone phosphate)

To 500 µL of water dissolving 10 mg of betamethasone phosphate was added 1,000 µL of 0.5 M zinc acetate aqueous solution. After centrifugation at 12,000 rpm for 5 minutes and removal of supernatant, the precipitate was cleaned by adding water, followed by centrifugation. The resultant precipitate and 1 mg of myristic acid and Tween 80 were dissolved (or suspended) in 1,000 µL of acetone, and added into water under stirring, to give primary nanoparticles. To this particle suspension, was added 1M calcium chloride aqueous solution (equimolar amount relative to myristic acid) and stirred for 30 minutes, followed by addition of 1M sodium hydrogen carbonate (0.2 times molar amount relative to calcium) and stirring for 1 to 12 hours, to give tertiary nanoparticles containing betamethasone phosphate. After preparation with various amounts of Tween 80, and centrifugation at 5,000 rpm for 5 minutes, particle size of supernatant particles and remaining amount of betamethasone phosphate (BP) were determined by HPLC. The results are shown in Table 14.

Effect of amount of Tween on particle formation

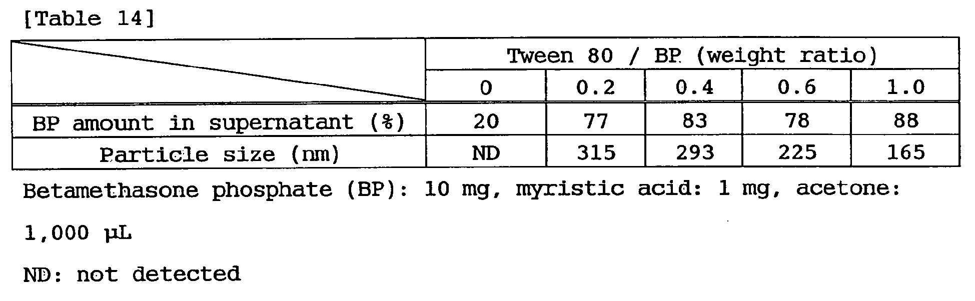

As is evident from the result of Table 14, when the preparation was made without mixing Tween 80, aggregates were formed, and BP was little detected in the supernatant. By mixing a certain amount of Tween 80, particles having excellent dispersion stability were prepared, and the larger the amount of Tween 80, the smaller particles could be prepared.

### Example 11: Preparation of retinol (vitamin A) particles

10 µL of a solution dissolving 6 mg of retinol (vitamin A) in ethanol or acetone, and 100 mg of soybean oil were mixed, and the mixture was added to a suspension of 22 mg of glycerin, 10 mg of lecithin, 10 mg of sodium oleate and 12 mg of oleic acid-polyethyleneglycol copolymer in water so that the total amount was 10 mL. The mixture was homogenized by using an ultrasonic wave generator or a French presser, to give primary nanoparticles containing retinol. Next, an equimolar amount of calcium chloride aqueous solution, relative to sodium oleate was added and stirred for an hour at room temperature, to give secondary nanoparticles.
Then, sodium hydrogen carbonate was added in a 0.2 to 1 time molar amount, relative to calcium chloride, followed by stirring for 3 hours to overnight, to give tertiary nanoparticles. The final retinol concentration was about 0.3 to 0.5%.

### Example 12: Preparation of retinol (vitamin A) particles

To 100 parts by weight of water, 0.5 parts by weight of sodium oleate was added, and the mixture was stirred by a stirrer until the sodium oleate was completely dissolved. Separately, 5.0 parts by weight of ethanol and 5.0 parts by weight of retinol 50C [product of BASF: mixture of 49% polyoxyethylene (20) sorbitan monolaurate (Tween 20); 47% retinol; 3% butylhydroxytoluene; 1% butylhydroxy anisole] were mixed and dissolved, and the resultant solution was added to the previous solution, and the mixture was stirred for 10 minutes by a stirrer, to give primary nanoparticles. Then 0.25 parts by weight of 1M calcium chloride aqueous solution was added and stirred by a stirrer for 10 minutes at room temperature, to give secondary nanoparticles. Then, 0.05 parts by weight of 1M sodium hydrogen carbonate aqueous solution was added and stirred overnight, to give tertiary nanoparticles. The particle size of nanoparticles obtained above was about 100 nm.

### Example 13: Preparation of ubidecarenone particles

To 1 g of soybean oil, 200 µL of 50 mg/ mL ubidecarenone solution in acetone was added and dissolved by stirring. To this solution, 4 mL of 25 mg/ mL lecithin solution in water was added and stirred. Further, 1 mL of 100 mg/ mL sodium oleate, 2 mL of 60 mg/mL Oleyl-O-PEG (SUNBRIGHT OE-020; NOF Corporation), 440 µL of 50% glycerin aqueous solution, and purified water were added to make the total volume of 10 mL. After stirring, emulsification was conducted using an ultrasonic wave generator (UD-201; TOMY SEIKO Co., Ltd.) to give a particle solution. Thereafter, 330 µL of 1M calcium chloride aqueous solution was added and mingled by rotation for 45 minutes, and further 330 µL of 1M sodium hydrogen carbonate aqueous solution was added and mingled by rotation for 45 minutes. Thereafter, excess metal salt and separate oil phase was removed by centrifugation to give tertiary nanoparticles of ubidecarenone.
Particle sizes of these particles were measured by using a particle size analyzer FRAR-1000 (OTSUKA ELECTRONICS CO., LTD.), and an average particle size was 276.6 nm.
Emulsification using a French press cell crusher (OMFA078A; Thermo IEC) also gave nanoparticles as well.
The tertiary nanoparticles of ubidecarenone thus obtained were left still for 5 days at 50°C without light shielding. No changes in appearance and particle size of particles were observed.

### Example 14: Production of ointments/hydrogels

Using the tertiary nanoparticles (encapsulating testosterone enanthate) obtained in Example 5, white vaseline, carboxymethylcellulose sodium and methyl paraoxybenzoate as appropriate, ointments and hydrogels were produced by mingling them until the entire mixture was homogenous.

### Example 15: Gel formulation

| Prescription: in 100 parts by weight of a hydrogel formulation | |
|---|---|
| Polyvinylpyrrolidone (Kollidon 90F) | 0.2 parts by weight |
| Disodium edentate | 0.1 part by weight |
| Polyvinyl alcohol (PVA) | 1.5 parts by weight |
| Benzalkonium chloride | 0.01 part by weight |
| Tertiary nanoparticles obtained in Example 12 | 0.1 part by weight |
| Deionized water | balance |

A gel agent was obtained from the above ingredients.

### Example 16: External patch (aqueous cataplasm)

### Prescription:

| Tertiary nanoparticles obtained in Example 5 | |
|---|---|
| (encapsulating testosterone enanthate) | 0.1 part by weight |
| Polyacrylic acid | 2.0 parts by weight |
| Sodium polyacrylate | 5.0 parts by weight |
| Carboxymethyl cellulose sodium | 2.0 parts by weight |
| Gelatin | 2.0 parts by weight |
| Polyvinylalcohol | 0.5 parts by weight |
| Glycerin | 25.0 parts by weight |
| Kaolin | 1.0 part by weight |
| Aluminum hydroxide | 0.6 parts by weight |
| Tartaric acid | 0.4 parts by weight |
| EDTA-2-sodium | 0.1 part by weight |
| Purified water | balance |

Using the above ingredients as a base, an external patch (aqueous cataplasms) was produced in a method well-known in the art.

### Example 17: Injectable agent

The tertiary nanoparticles (encapsulating cyclosporine A) obtained in Example 5 was dissolved in distilled water for injection, and configured to contain a tonicity agent. After adjusting pH at 6.9, the resultant solution was packed in a vial which was subjected to high-pressure and high-temperature sterilization, to give an injectable agent.

### INDUSTRIAL APPLICABILITY

As described above, the present invention provides drug-containing nanoparticles (secondary nanoparticles) provided by causing primary nanoparticles containing a fat-soluble drug or fat-solubilized water-soluble drug to act with a bivalent or trivalent metal salt, drug-containing nanoparticles (tertiary nanoparticles) provided by first causing primary nanoparticles containing a fat-soluble drug or fat-solubilized water-soluble drug to act with a bivalent or trivalent metal salt to thereby obtain secondary nanoparticles and thereafter causing a monovalent to trivalent basic salt to act on the secondary nanoparticles, as well as a process for producing these nanoparticles, and a transdermal or transmucosal external preparation or injectable preparation in which these nanoparticles are contained. Nanoparticles of the present invention have a revolutionary effect of enabling transdermal or transmucosal in vivo absorption of fat-soluble drugs and water-soluble drugs, which was not satisfactorily attained hitherto, and provide an external preparation or injectable preparation containing a fat-soluble/water-soluble drug and realizing high absorptivity and sustained-releasability.

## Claims

1. Drug-containing nanoparticles provided by causing primary nanoparticles containing a fat-soluble drug or a fat-solubilized water-soluble drug to act with a bivalent or trivalent metal salt.

2. Drug-containing nanoparticles provided by causing primary nanoparticles containing a fat-soluble drug or fat-solubilized water-soluble drug to act with a bivalent or trivalent metal salt to give secondary nanoparticles, and causing a monovalent to trivalent basic salt to act with the secondary nanoparticles.

3. The drug-containing nanoparticles according to claim 1 or 2, wherein the primary nanoparticles are produced by causing the fat-soluble drug or the fat-solubilized water-soluble drug, a medium- or long-chain organic compound having a negative ion residue and a surfactant to act with each other.

4. The drug-containing nanoparticles according to claim 3, wherein the medium- or long-chain organic compound having a negative ion residue is a C₆-C₂₄ fatty acid or its salt.

5. The drug-containing nanoparticles according to claim 4, wherein the C₆-C₂₄ fatty acid is selected from unsaturated fatty acids such as oleic acid, linoleic acid, and linolenic acid, and saturated fatty acids such as lauric acid, myristic acid, and palmitic acid.

6. The drug-containing nanoparticles according to claim 1 or 2, wherein the bivalent or trivalent metal salt is a calcium salt, a zinc salt, an iron salt, or a copper salt.

7. The drug-containing nanoparticles according to claim 2, wherein the monovalent to trivalent basic salt is selected from hydrogen carbonates, hydrogen phosphates, carbonates, phosphates, oxalates, lactates, and urates.

8. The drug-containing nanoparticles according to claim 1 or 2, wherein fat-solubilization of water-soluble drug is carried out by contact between the water-soluble drug and the bivalent or trivalent metal ion, contact between the water-soluble drug and an acidic or basic polysaccharide, or adjustment of pH or change in ion strength of the solution in which the water-soluble drug is dissolved.

9. The drug-containing nanoparticles according to claim 8, wherein the bivalent or trivalent metal ion to be brought into contact with the water-soluble drug is selected from a zinc ion, a calcium ion, an iron ion, and a copper ion.

10. The drug-containing nanoparticles according to claim 3 or 9, wherein the surfactant is one or more selected from glycerin, lecithin, polyoxyethylene (20) sorbitan monooleate (Tween 80), polyoxyethylene (20) sorbitan monolaurate (Tween 20), polyoxyethylene (20) sorbitan monostearate (Tween 60), polyoxyethylene (20) sorbitan monoparmitate (Tween 40), polyoxyethylene (20) sorbitan trioleate (Tween 85), polyoxyethylene (8) octylphenyl ether, polyoxyethylene (20) cholesterol ester, lipid-polyethylene glycol, polyoxyethylene hydrogenated castor oil, and fatty acid-polyethylene glycol copolymer.

11. The drug-containing nanoparticles according to any one of claims 1 to 10, wherein the fat-soluble drug or water-soluble drug is a chemical compound that has a molecular weight of 1,000 or less, exhibits bioactivity and are applicable to human.

12. The drug-containing nanoparticles according to claim 11, wherein the fat-soluble drug is insoluble to poorly soluble to water and soluble to organic solvents.

13. The drug-containing nanoparticles according to claim 11 or 12, wherein the fat-soluble drug is selected from steroid hormones, immuno suppressing or modulating agents, anticancer agents, antibiotics, chemotherapeutic agents, antiviral agents, non-steroidal anti-inflammatory agents, antipsychotic agents, calcium antagonists, antihypertensive agents, prostaglandin drugs, and lipophilic vitamins.

14. The drug-containing nanoparticles according to any one of claims 11 to 13, wherein the fat-soluble drug is selected from testosterone enanthate, testosterone propionate, testosterone, estradiol, estradiol valerate, estradiol benzoate, dexamethasone acetate, betamethasone, betamethasone dipropionate, betamethasone valerate, prednisolone acetate, cyclosporine, tacrolimus, paclitaxel, irinotecan hydrochloride, cisplatin, methotrexate, carmofur, tegafur, doxorubicin, clarithromycin, aztreonam, cefdinir, nalidixic acid, ofloxacin, norfloxacin, ketoprofen, flurbiprofen, flurbiprofen axetil, chlorpromazine, diazepam, nifedipine, nicardipine hydrochloride, amlodipine besilate, candesartan cilexetil, aciclovir, vidarabine, efavirenz, alprostadil, dinoprostone, ubidecarenone, vitamin A (retinol), vitamin D, vitamin E, and vitamin K.

15. The drug-containing nanoparticles according to claim 11, wherein the water-soluble drug is a drug that is fat-solubilized by binding with a bivalent or trivalent metal ion.

16. The drug-containing nanoparticles according to claim 11 or 15, wherein the water-soluble drug is selected from water-soluble steroid hormones, immuno suppressing or modulating agents, anticancer agents, antibiotics, chemotherapeutic agents, antiviral agents, non-steroidal anti-inflammatory agents, antipsychotic agents, antihypertensive agents, prostaglandin drugs, and vitamins.

17. The drug-containing nanoparticles according to claim 11, 15, or 16, wherein the water-soluble drug is selected from betamethasone phosphate, dexamethasone phosphate, prednisolone phosphate, prednisolone succinate, hydrocortisone succinate, vancomycin, vincristine, vinplastin chloramphenicol succinate, latamoxef, cefpirome, carumonam, clindamycin phosphate, and abacavir.

18. The drug-containing nanoparticles according to claim 11, wherein the fat-soluble drug is testosterone enanthate, cyclosporine, betamethasone valerate, ubidecarenone, or vitamin A (retinol), and the water-soluble drug is betamethasone phosphate.

19. The drug-containing nanoparticles according to any one of claims 1 to 18, wherein the particles have a diameter ranging from 1 to 200 nm.

20. A transdermal or transmucous external preparation comprising the drug-containing nanoparticles according to any one of claims 1 to 19.

21. The external preparation according to claim 20, wherein the external preparation is selected from ointments, gels, sublingual tablets, buccal tablets, liquids and solutions, sprays for buccal/lower respiratory tract, inhalations, suspensions, hydrogels, lotions, cataplasms, and patches.

22. An injectable preparation comprising the drug-containing nanoparticles according to any one of claims 1 to 19.

23. A process of producing drug-containing nanoparticles comprising; dissolving a fat-soluble drug or fat-solubilized water-soluble drug, a medium- or long-chain organic compound having a negative ion residue, and a surfactant in an organic solvent or a water-containing organic solvent to give a solution; dispersing the solution in water to produce primary nanoparticles; and causing a bivalent or trivalent metal salt to act with the solution containing the primary nanoparticles.

24. A process of producing drug-containing nanoparticles comprising; dissolving a fat-soluble drug or fat-solubilized water-soluble drug, a medium- or long-chain organic compound having a negative ion residue and a surfactant in an organic solvent or a water-containing organic solvent to give a solution; dispersing the solution in water to produce primary nanoparticles; causing a bivalent or trivalent metal salt to act with the solution containing the primary nanoparticles to produce secondary nanoparticles; and causing a monovalent to trivalent basic salt to act with the secondary nanoparticles.

25. The production process according to claim 23 or 24, wherein the organic solvent is one or more selected from acetone, ethanol, propanol, and butanol.

26. The production process according to claim 23 or 24, wherein fat-solubilization of the water-soluble drug comprises bringing the water-soluble drug into contact with the bivalent or trivalent metal ion.
